# EUROPEAN PATENT APPLICATION

(11) **EP 3 753 546 A1**
(43) Date of publication of application: **23.12.2020**
(21) Application number: 19754637.7
(22) Date of filing: 01.02.2019
(51) Int. Cl.: A61K 8/02, A61K 8/19, A61K 8/25, A61K 8/29, A61K 8/31, A61K 8/81, A61K 8/891, A61K 8/892, A61Q 1/02, A61Q 1/08, A61Q 1/10, A61Q 1/12

(54) **SOLID POWDER COSMETIC**

(30) Priority: 14.02.2018 JP 2018024280
(71) Applicant: Tokiwa Corporation, Nakatsugawa-shi, Gifu 508-8555 (JP)
(72) Inventor: MIYAZAKI Mai, Kawaguchi-shi, Saitama 332-0017 (JP)
(74) Representative: Michalski Hüttermann & Partner Patentanwälte mbB
(86) International application number: PCT/JP2019/003697
(87) International publication number: WO 2019/159722

(57) **Abstract**

A solid powder cosmetic is a solid powder cosmetic containing a powder component and an oily component, the content of the oily component is 5% to 25% by mass based on the total amount of the solid powder cosmetic, and the oily component contains: (A) a silicone compound whose viscosity at 25°C is 4,000 to 100,000 mPa·s and (B) an oil agent that is in a semi-solid state at 25°C.

## Description

### Technical Field

The present invention relates to a solid powder cosmetic.

### Background Art

A solid powder cosmetic is composed of a powder component and an oily component, and since solid powder cosmetics are frequently used in the form of being stored in compact vessels, impact resistance is required in consideration of the transportation upon carrying around. Furthermore, since a solid powder cosmetic that is used as a makeup cosmetic such as an eyeshadow, a blusher, or a foundation is used by scraping the surface of the cosmetic using an applicator such as a brush or a sponge and applying the cosmetic on the skin, it is desirable that the condition of being picked up by a small tool, the smoothness of spreading upon application, and the feeling of use such as adhesion and high compatibility with the skin, are satisfactory.

As a technology for enhancing the impact resistance of a solid powder cosmetic, for example, in the following Patent Literature 1, a solid powder cosmetic obtained by blending a hollow resin powder having a particular specific gravity and a silica having a particular oil absorption as powder components, has been proposed. Furthermore, in the following Patent Literature 2, a powdery solid makeup cosmetic obtained by blending a high-viscosity dimethyl polysiloxane and a powder with a high aspect ratio has been proposed as a solid powder cosmetic having smooth usability. Furthermore, in the following Patent Literature 3, for the purpose of improving the impact resistance, picking up by an applicator, and the feeling of use, a solid powder cosmetic obtained by blending an organic spherical powder and a particular non-silicone oil in their respective predetermined amounts, and further blending the resultant with a partially crosslinkable organopolysiloxane polymerization product, has been proposed.

### Citation List

### Patent Literature

Patent Literature 1: Japanese Unexamined Patent Publication No. 2013-227277
Patent Literature 2: Japanese Unexamined Patent Publication No. H9-249531
Patent Literature 3: Japanese Unexamined Patent Publication No. 2013-227282

### Summary of Invention

### Technical Problem

However, the solid powder cosmetic described in Patent Literature 1 is improved in view of impact resistance such as drop resistance; however, since the solid powder cosmetic contains a large amount of oily components, the cosmetic hardens over time, and caking by which the usability such as picking-up is deteriorated tends to occur easily. On the other hand, the powdery solid makeup cosmetic described in Patent Literature 2 has insufficient drop resistance and has a problem that two kinds of powder flying such as the scattering of a powder remaining without attaching to an applicator when the cosmetic is scraped by the applicator, and of a powder that has fallen without adhering to the skin when the applicator is brought into contact with the skin.

Furthermore, the solid powder cosmetic described in Patent Literature 3 is likely to have the occurrence of the above-described problem of powder flying and is not satisfactory even for the feeling of use such as adherence to the skin.

An object of the present invention is to provide a solid powder cosmetic which has satisfactory feeling of use and sufficient drop resistance, in which caking is sufficiently suppressed and picking up by an applicator is satisfactory, and which can sufficiently suppress powder flying.

### Solution to Problem

In order to solve the above-described problems, the inventors of the present invention conducted a thorough investigation, and as a result, the inventors found that in a solid powder cosmetic containing an oily component at a particular proportion, when two particular oily components are blended as the oily component, the above-described requirements can all be satisfied, thus completing the present invention.

That is, the present invention provides a solid powder cosmetic containing a powder component and an oily component, in which the content of the oily component is 5% to 25% by mass based on the total amount of the solid powder cosmetic, and the oily component contains: (A) a silicone compound whose viscosity at 25°C is 4,000 to 100,000 mPa·s, and (B) an oil agent that is in a semi-solid state at 25°C.

According to the solid powder cosmetic of the present invention, when the above-described component (A) and component (B) are blended with the oily component in a particular amount, the solid powder cosmetic has satisfactory feeling of use and sufficient drop resistance, caking is sufficiently suppressed, picking up by an applicator is satisfactory, and also, the above-described two types of powder flying can be sufficiently suppressed.

Furthermore, the solid powder cosmetic of the present invention can be satisfactorily formed by any method of wet production methods and dry production methods and can be a cosmetic with excellent color development.

With regard to the solid powder cosmetic of the present invention, from the viewpoint of further enhancing the absence of powder flying, the picking up by an applicator, and the feeling of use, it is preferable that the component (A) includes one or more kinds of silicone compounds selected from the group consisting of dimethyl polysiloxane, diphenyl polysiloxane, and dimethiconol.

Furthermore, from the viewpoint of enhancing the drop resistance and adherence, it is preferable that the component (B) includes one or more kinds of oil agents selected from the group consisting of a pentaerythritol fatty acid ester, petrolatum, a dimer acid ester, a dimer diol derivative, a phytosterol fatty acid ester, and a cholesterol fatty acid ester.

With regard to the solid powder cosmetic of the present invention, it is preferable that the content of the component (A) is 0.5% to 15.0% by mass based on the total amount of the solid powder cosmetic, and the content of the component (B) is 0.1% to 10.0% by mass based on the total amount of the solid powder cosmetic.

### Advantageous Effects of Invention

According to the present invention, a solid powder cosmetic which has satisfactory feeling of use and sufficient drop resistance, in which caking is sufficiently suppressed and the picking up by an applicator is satisfactory, and which can sufficiently suppress powder flying, can be provided.

### Description of Embodiments

A solid powder cosmetic of the present embodiment contains a powder component and an oily component.

Regarding the powder component, any powder that is usually used for cosmetics can be used without any particular limitations, and for example, an extender powder, a white pigment, a colored pigment, and the like may be mentioned. The shape of the powder is also not particularly limited, and the powder may have a shape such as a spherical shape, a plate shape, or a needle shape; a particle size of an aerosol form, a microparticulate grade, or a pigment grade; and a particle structure such as porousness or non-porousness.

Specific examples include extender pigments such as mica, synthetic mica, sericite, talc, kaolin, silicon carbide, barium sulfate, bentonite, smectite, aluminum oxide, silica, magnesium oxide, zirconium oxide, magnesium carbonate, calcium carbonate, chromium oxide, and aluminum magnesium hydroxide; white pigments such as titanium oxide and zinc oxide; organic powders such as a nylon powder, a polymethyl methacrylate powder, an acrylonitrile-methacrylic acid copolymer powder, a vinylidene chloride-methacrylic acid copolymer, a polyethylene powder, a polystyrene powder, an organopolysiloxane elastomer powder, a polymethylsilsesquioxane powder, a urethane powder, a wool powder, a silk powder, a cellulose powder, and an N-acyl lysine powder; composite powders such as microparticulate titanium oxide-coated mica-titanium, microparticulate titanium oxide-coated nylon, barium sulfate-coated mica-titanium, titanium oxide-containing silica, and zinc oxide-containing silica; and metal soaps such as magnesium stearate, zinc myristate, aluminum stearate, and calcium stearate.

Examples of the colored pigment include inorganic colored pigments such as colcothar, yellow iron oxide, black iron oxide, cobalt oxide, chromium oxide, Ultramarine Blue, Prussian Blue, titanium oxide, and zinc oxide; organic colored pigments such as aluminum lakes Red No. 228, Red No. 226, Blue No. 404, Red No. 202, and Yellow No. 4; pearl pigments such as mica-titanium, microparticulate titanium oxide-coated mica-titanium, barium sulfate-coated mica-titanium, fish scale guanine, bismuth oxychloride, and aluminum flakes; natural coloring materials such as carmine and safflower; and the like.

These powder components are preferably hydrophobic-treated powders from the viewpoints of color development and adherence. Examples of the hydrophobic treatment include higher fatty acids, a metal soap, fats and oils, a wax, a silicone compound, a fluorine compound, a surfactant, and a dextrin fatty acid ester. According to the present embodiment, from the viewpoints of the feeling of use and color development, a metal soap treatment is preferred, and a magnesium stearate treatment is more preferred.

The powder components can be used singly or in combination of two or more kinds thereof.

The average particle size of the powder component can be adjusted to 1 to 200 µm, and can be adjusted to 5 to 150 µm.

With regard to the solid powder cosmetic of the present embodiment, from the viewpoint that the drop resistance and adherence can be further enhanced, it is preferable that the powder component includes a plate-shaped powder.

Regarding the plate-shaped powder, from the viewpoint of suppressing powder flying, the aspect ratio is preferably 10 to 200, and more preferably 20 to 100. The aspect ratio means average particle size/average thickness.

Regarding the plate-shaped powder, for example, talc, mica, synthetic mica, sericite, silicic anhydride, kaolin, calcium carbonate, boron nitride, and photoluminescent powders can be used.

The content of the plate-shaped powder in the solid powder cosmetic of the present embodiment is preferably 30% to 90% by mass, and more preferably 40% to 85% by mass, based on the total amount of the solid powder cosmetic. By adjusting the content of the plate-shaped powder to the above-described range, the drop resistance and adherence can be further enhanced.

Regarding the solid powder cosmetic of the present embodiment, from the viewpoints of the suppression of powder flying and the drop resistance, the content of a spherical powder is preferably less than 5% by mass, more preferably 3% by mass or less, and even more preferably 1% by mass or less, based on the total amount of the solid powder cosmetic.

With regard to the solid powder cosmetic of the present embodiment, from the viewpoint that the drop resistance can be further enhanced, it is preferable that the powder component includes a metal soap.

Regarding the metal soap, those mentioned above can be used, and from the viewpoint of the drop resistance, aluminum distearate and zinc myristate are preferred.

The content of the metal soap in the solid powder cosmetic of the present embodiment is preferably 0.1% to 10% by mass, and more preferably 1% to 8% by mass, based on the total amount of the solid powder cosmetic. By adjusting the content of the metal soap to the above-described range, it becomes easy to realize a solid powder cosmetic in which cracks are not easily generated even by transportation upon carrying around, and the impact resistance is excellent.

The content of the powder component in the solid powder cosmetic of the present embodiment is preferably 75% to 95% by mass, and more preferably 80% to 95% by mass, based on the total amount of the solid powder cosmetic.

The solid powder cosmetic of the present embodiment can contain an oily component in an amount of 5% to 25% by mass based on the total amount of the solid powder cosmetic, and it is preferable that the solid powder cosmetic contains the oily component in an amount of 5% to 20% by mass.

It is preferable that the oily component includes: (A) a silicone compound whose viscosity at 25°C is 4,000 to 100,000 mPa·s (may also be referred to as component (A)), and (B) an oil agent that is in a semi-solid state at 25°C (may also be referred to as component (B)).

By incorporating the above-described component (A) and component (B) in combination as an oily component into the solid powder cosmetic in which an oily component is included, the solid powder cosmetic has satisfactory feeling of use and sufficient drop resistance, caking is sufficiently suppressed, picking up by an applicator is satisfactory, and a problem of two kinds of powder flying such as the scattering of a powder remaining without attaching to an applicator when the cosmetic is scraped by the applicator, and of a powder that has fallen without adhering to the skin when the applicator is brought into contact with the skin, can be sufficiently suppressed. The solid powder cosmetic can be satisfactorily formed by any method of wet production methods and dry production methods and can be a cosmetic with excellent color development.

Examples of the component (A) include dimethyl polysiloxane, diphenyl polysiloxane, and dimethiconol. Regarding the component (A), one kind thereof can be used alone, or two or more kinds thereof can be used in combination.

Furthermore, from the viewpoints of the suppression of powder flying and the formability, the component (A) is preferably a silicone compound whose viscosity at 25°C is 4,000 to 100,000 mPa·s, and more preferably a silicone compound whose viscosity is 9,000 to 50,000 mPa·s. When the viscosity of the silicone compound is in the above-described range, a balance between the suppression of powder flying and satisfactory formability with difficulties in the occurrence of caking is easily achieved.

Meanwhile, the viscosity is measured, in a case in which the viscosity of the component (A) is 1,000 to 10,000 mPa·s, using a BH type viscometer and a rotor No. 3 at a speed of rotation of 10 rpm; in a case in which the viscosity is 10,000 to 100,000 mPa·s, the viscosity is measured using a BH type viscometer and a rotor No. 6 at a speed of rotation of 10 rpm; and in a case in which the viscosity is 100,000 to 800,000 mPa·s, the viscosity is measured using a BH type viscometer and a rotor No. 7 under the conditions of a speed of rotation of 4 rpm, at 25°C. Incidentally, regarding the viscometer and the rotor, those manufactured by Toki Sangyo Co., Ltd. can be used.

From the viewpoint of suppressing powder flying and enhancing the feeling of use, the content of the component (A) in the solid powder cosmetic of the present embodiment is preferably 0.5% to 15.0% by mass, more preferably 1.0% to 10.0% by mass, and even more preferably 2.0% to 7.0% by mass, based on the total amount of the solid powder cosmetic.

The oil agent that is in a semi-solid state at 25°C, which is the component (B), is a semi-solid oily component that exhibits high viscosity at 25°C. The oil agent refers to an oily component, for which the melting point is measured to be higher than 25°C by General Testing Method, Melting Point Measurement Method (second method) described in the Japanese Standards of Quasi-drug Ingredients 2006 (published by Yakuji Nippo, Ltd.).

Examples of the component (B) include petrolatum, a dimer acid ester, a dimer diol derivative, a phytosterol fatty acid ester, a cholesterol fatty acid ester, and a pentaerythritol fatty acid ester.

Regarding the petrolatum, commercially available products such as CROLATUM V (manufactured by Croda Japan K.K.), NOMCORT W (manufactured by The Nisshin Oillio Group, Ltd.), and SUNWHITE P-150 (manufactured by Nikko Rica Corporation) can be used.

Examples of the dimer acid ester include (phytosteryl/isotearyl/cetyl/stearyl/behenyl) dimer dilinoleate, bis(behenyl/isostearyl/phytostearyl) dimer dilinoleyl dimer dilinoleate, bis(phytosteryl/isostearyl/cetyl/stearyl/behenyl) dimer dilinoleyl dimer dilinoleate, and the like. Regarding the dimer acid ester, for example, commercially available products such as Plandool-G and Plandool-H (all manufactured by NIPPON FINE CHEMICAL CO., LTD.) can be used.

Examples of the dimer diol derivative include a hydroxyalkyl (C12-14) hydroxy dimer dilinoleyl ether, a hydroxyalkyl (C16-18) hydroxydimer dilinoeyl ether, and the like. Regarding the dimer diol derivative, for example, commercially available products such as SUPERMOL LM and SUPERMOL PS (all manufactured by Croda International plc) can be used.

Examples of the phytosterol fatty acid ester include macadamia nut fatty acid phytosteryl, phytosteryl oleate, bis(behenyl/isostearyl/phytosteryl) dimer dilinoleyl dimer dilinoleic acid, and (phytosteryl/isostearyl/cetyl/stearyl/behenyl) dimer dilinoleate. Regarding the phytosterol fatty acid ester, commercially available products such as SALACOS PO (manufactured by The Nisshin Oillio Group, Ltd.), YOFCO MAS, Plandool-G, Plandool-S, and Plandool-H (all manufactured by NIPPON FINE CHEMICAL CO., LTD.) can be used.

Examples of the cholesterol fatty acid ester include a fatty acid (C10-30) (cholesterol/lanosterol) ester and cholesteryl hydroxystearate. Regarding the cholesterol fatty acid ester, commercially available products such as SUPER STEROL ESTER (manufactured by Croda Japan K.K.) and SALACOS HS (manufactured by The Nisshin Oillio Group, Ltd.) can be used.

Examples of the pentaerythritol fatty acid ester include dipentaerythrityl hexahydroxystearate, dipentaerythrityl (hydroxystearate/isostearate), dipentaerythrityl (hydroxystearate/stearate/rosinate), dipentaerythrityl hexa(hydroxystearate/stearate/rosinate), and dipentaerythrityl tetra(hydroxystearate/isostearate). Regarding the pentaerythritol fatty acid ester, commercially available products such as COSMOL 168M, COSMOL 168E, COSMOL 168AR, COSMOL 168ARV, and COSMOL EV (manufactured by The Nisshin Oillio Group, Ltd.) can be used.

The component (B) is preferably a pentaerythritol fatty acid ester, from the viewpoint of suppressing powder flying.

From the viewpoint of the drop resistance, the content of the component (B) in the solid powder cosmetic of the present embodiment is preferably 0.1% to 10.0% by mass, more preferably 0.5% to 8.0% by mass, and even more preferably 1.0% to 5.0% by mass, based on the total amount of the solid powder cosmetic.

The total content of the component (A) and the component (B) in the solid powder cosmetic of the present embodiment is preferably 2.0% to 20.0% by mass, more preferably 2.5% to 15.0% by mass, and even more preferably 3.0% to 12.0% by mass, based on the total amount of the solid powder cosmetic.

With regard to the solid powder cosmetic of the present embodiment, the ratio (A/B) of the content A of the component (A) and the content B of the component (B) is preferably 1/20 to 150/1, more preferably 1/8 to 20/1, and even more preferably 2/5 to 7/1.

Regarding other oily components other than the component (A) and the component (B), for example, any oily component that is usually used for cosmetic products can be used without any particular limitations, and regardless of the origin such as an animal oil, a plant oil, or a synthetic oil, and of the characteristics such as a solid oil, a semi-solid oil, a liquid oil, or a volatile oil, fats and oils, hardened oils, fatty acids, higher alcohols, ester oils, hydrocarbon oils, silicone oils, fluorine-based oils, lanolin derivatives, oily gelling agents, and the like can be used.

The solid powder cosmetic of the present embodiment can contain, in addition to the above-described components, components that are usually used in cosmetics, for example, a surfactant, an antiseptic agent, an antioxidant, a coloring material, a thickening agent, a pH adjusting agent, a fragrance, an ultraviolet absorber, an ultraviolet scattering agent, a chelating agent, an anti-inflammatory agent, a humectant, and the like.

The solid powder cosmetic of the present embodiment is suitable as a makeup cosmetic for a foundation, a facial powder, a facial color, an eyeshadow, an eyebrow pencil, a blusher, and the like.

Next, a method for producing a solid powder cosmetic of the present embodiment will be described.

The method for producing a solid powder cosmetic of the present embodiment includes a step of mixing a cosmetic base material containing a powder component and an oily component with a dispersing medium and preparing a slurry; and a step of compression-molding and drying the slurry charged into a vessel.

Regarding the powder component and the oily component, those mentioned above may be mentioned, and the blending amounts in a cosmetic base material can also be adjusted similarly to the above-mentioned preferred ranges in the solid powder cosmetic. Furthermore, the composition other than the powder component and the oily component of the cosmetic base material can also be adjusted similarly to the above-mentioned preferred composition of the solid powder cosmetic.

The cosmetic base material according to the present embodiment can be prepared by, for example, a method including a step of obtaining a first mixture mixed with a powder component; a step of obtaining a second mixture mixed with an oily component; and a step of mixing the first mixture and the second mixture.

The step of obtaining a first mixture may be carried out using, for example, a Super Mixer, a Henschel Mixer, or the like, and if necessary, pulverization may be carried out using an atomizer or the like.

The step of obtaining a second mixture can be carried out using, for example, a Disper, a Homo Mixer, or the like, and the oily component can be mixed while heating at 60°C to 80°C, and preferably 60°C to 70°C.

The step of mixing the first mixture and the second mixture can be carried out using, for example, a Super Mixer, a Henschel Mixer, or the like, and if necessary, pulverization may be carried out using an atomizer or the like.

For the preparation of a slurry, a method of adding a dispersing medium to the cosmetic base material obtainable as described above and mixing these, may be mentioned.

As the dispersing medium, a volatile solvent can be used. Examples of the volatile solvent include light liquid isoparaffin, ethyl alcohol, acetone, isopropyl alcohol, and water.

The blending proportions of the cosmetic base material and the dispersing medium can be adjusted to cosmetic base material : dispersing medium = 100 : 60 to 100 : 10 as a mass ratio, and from the viewpoint of formability, 100 : 40 to 100 : 20 is preferred.

Regarding the mixing of the cosmetic base material and the dispersing medium, for example, a method of kneading using a kneader, a universal stirrer, or the like may be mentioned. Furthermore, if necessary, mixing can be performed while heating.

In the step of compression-molding and drying the slurry charged into a vessel, the slurry obtained as described above is degassed as necessary and charged into a predetermined vessel, subsequently this is compression-molded by suction compression-molding or the like, and then the slurry can be dried appropriately by means of a dryer.

Examples of the predetermined vessel include an inner tray such as a metal tray or a resin tray, and the like.

The solid powder cosmetic according to the present embodiment is obtained through the above-described processes.

The method for producing a solid powder cosmetic of the present embodiment may be a dry production method. For example, a cosmetic base material containing a powder component and an oily component is prepared similarly to the above-described wet production method, this cosmetic base material is charged into a predetermined vessel and is compression-molded, and thereby a solid powder cosmetic can be obtained.

### Examples

Hereinafter, the present invention will be described in more detail by way of Examples; however, the technical scope of the present invention is not intended to be limited by these Examples. Meanwhile, the numerical values in the tables represent the contents (% by mass) based on the total amount of the cosmetic base material (sum of components other than a volatile solvent). With regard to a volatile solvent, the numerical value represents the proportion (parts by mass) with respect to 100 parts by mass of the total amount of the cosmetic base material (sum of components other than the volatile solvent).

Prior to Examples, the evaluation methods employed in the various Examples will be described.

### (1) Absence of powder flying

In order to evaluate powder flying at the time of being picked up by an applicator or at the time of application, a sample of a solid powder cosmetic was reciprocatingly stroked 10 times with a brush, and the amount of powder that fell out without attaching was observed by visual inspection. A 4-grade evaluation was carried out according to the following determination criteria, each sample was assigned with a score, and the average point of n = 5 was determined according to the following criteria.

### [Evaluation criteria]

4: Powder flying almost does not occur.
3: Powder flying occurs slightly.
2: Powder flying occurs.
1: Powder flying occurs severely.

### [Evaluation criteria (average point of scores)]

A: 3.5 or more
B: 3.0 or more and less than 3.5
C: 2.0 or more and less than 3.0
D: Less than 2.0

### (2) Picking up by applicator

Ten expert panels for cosmetic product evaluation were asked to use samples of solid powder cosmetics with a brush, and for the "picking up by applicator", each of the panels carried out a 7-grade evaluation according to the following evaluation criteria and assigned a score to each sample. Furthermore, the average points of the scores of all the panels were determined according to the following criteria.

### [Evaluation criteria]

6: Very satisfactory
5: Satisfactory
4: Slightly satisfactory
3: Ordinary
2: Slightly poor
1: Poor
0: Very poor

### [Determination criteria (average point of scores)]

A: 5.0 or more
B: 3.5 or more and less than 5.0
C: 1.5 or more and less than 3.5
D: Less than 1.5

### (3) Absence of caking

Ten expert panels for cosmetic product evaluation were asked to use samples of solid powder cosmetics with a brush, and for the "absence of caking", each of the panels carried out a 7-grade evaluation according to the following evaluation criteria and assigned a score to each sample. Furthermore, the average points of the scores of all the panels were determined according to the following criteria.

### [Evaluation criteria]

6: Very satisfactory
5: Satisfactory
4: Slightly satisfactory
3: Ordinary
2: Slightly poor
1: Poor
0: Very poor

### [Determination criteria (average point of scores)]

A: 5.0 or more
B: 3.5 or more and less than 5.0
C: 1.5 or more and less than 3.5
D: Less than 1.5

### (4) Feeling of use (spreading, adherence, good skin compatibility)

Ten expert panels for cosmetic product evaluation were asked to use samples of solid powder cosmetics with a brush, and for the "feeling of use (spreading, adherence, and good skin compatibility)", each of the panels carried out a 7-grade evaluation according to the following evaluation criteria and assigned a score to each sample. Furthermore, the average points of the scores of all the panels were determined according to the following criteria.

### [Evaluation criteria]

6: Very satisfactory
5: Satisfactory
4: Slightly satisfactory
3: Ordinary
2: Slightly poor
1: Poor
0: Very poor

### [Determination criteria (average point of scores)]

A: 5.0 or more
B: 3.5 or more and less than 5.0
C: 1.5 or more and less than 3.5
D: Less than 1.5

### (5) Good color development

Ten expert panels for cosmetic product evaluation were asked to use samples of solid powder cosmetics with a brush, and for the "good color development", each of the panels carried out a 7-grade evaluation according to the following evaluation criteria and assigned a score to each sample. Furthermore, the average points of the scores of all the panels were determined according to the following criteria.

### [Evaluation criteria]

6: Very satisfactory
5: Satisfactory
4: Slightly satisfactory
3: Ordinary
2: Slightly poor
1: Poor
0: Very poor

### [Determination criteria (average point of scores)]

A: 5.0 or more
B: 3.5 or more and less than 5.0
C: 1.5 or more and less than 3.5
D: Less than 1.5

### (6) Formability

For a molded article obtained by performing compression-molding and drying, the presence or absence of cracking, flaking, fissures, and the like occurring on the surface of samples of solid powder cosmetics was observed by visual inspection, and a 4-grade evaluation of the level of occurrence was carried out according to the following determination criteria. Each of the samples was assigned with a score, and the average point of n = 5 was determined according to the following criteria.

### [Evaluation criteria]

4: No change
3: The occurrence of cracking, peeling, fissures, and the like is slightly recognized.
2: The occurrence of cracking, peeling, fissures, and the like is recognized.
1: The frequency of occurrence of cracking, peeling, fissures, and the like is very high.

### [Determination criteria (average point of scores)]

A: 3.5 or more
B: 3.0 or more and less than 3.5
C: 2.0 or more and less than 3.0
D: Less than 2.0

### (7) Impact resistance

Samples of solid powder cosmetics were dropped five times from a height of 50 cm on a P tile in a direction in which the contents face upward, the surface state was observed, and a 4-grade evaluation was carried out on the occurrence of cracking, chipping, collapse, and the like according to the following determination criteria. Each of the samples was assigned with a score, and the average point of n = 5 was determined according to the following criteria.

### [Evaluation criteria]

4: No change
3: Cracking is recognized.
2: Chipping is recognized.
1: Collapse of a half or more is recognized.

### [Determination criteria (average point of scores)]

A: 3.5 or more
B: 3.0 or more and less than 3.5
C: 2.0 or more and less than 3.0
D: Less than 2.0

### (Examples 1 to 18 and Comparative Examples 1 to 6)

Blushers having the compositions shown in Tables 1 to 4 were prepared by the following production method, and the above-described evaluations were carried out. The results are presented together in Tables 1 to 4.

### <Production method>

Powder components were uniformly dispersed with a Henschel Mixer, and mixture I was obtained. Oily components and other components were heated to 70°C and uniformly dispersed, and mixture II was obtained. While mixture I was stirred with a Henschel Mixer, mixture II was added thereto, the mixture was uniformly dispersed, and a cosmetic base material was obtained. To 100 parts by mass of this cosmetic base material, light liquid isoparaffin in an amount of the parts by mass indicated in the table was added as a volatile solvent, the mixture was kneaded with a kneading machine, and thereby a slurry was obtained. The slurry thus obtained was charged into a metal tray and was compression-molded, and then the solvent was removed by drying the slurry. Thereby, a sample of a blusher was produced.

**[Table 1]**

| | | | Example | | | | | | |
|---|---|---|---|---|---|---|---|---|---|
| | | | 1 | 2 | 3 | 4 | 5 | 6 | 7 |
| Oily component | Component (A) | Silicone compound A | 4.00 | - | - | - | - | - | - |
| | | Silicone compound B | - | 4.00 | - | - | - | 3.00 | 4.00 |
| | | Silicone compound C | - | - | 4.00 | - | - | - | - |
| | | Silicone compound D | - | - | - | 4.00 | - | - | - |
| | | Silicone compound E | - | - | - | - | 4.00 | - | - |
| | Component (B) | Oil agent A | 3.50 | 3.50 | 3.50 | 3.50 | 3.50 | 1.00 | 3.50 |
| | Others | Ethylhexyl stearate | 6.00 | 6.00 | 6.00 | 6.00 | 6.00 | 1.00 | 17.50 |
| Other components | Sorbitan isostearate | | 0.10 | 0.10 | 0.10 | 0.10 | 0.10 | 0.10 | 0.10 |
| | Tocopherol | | 0.02 | 0.02 | 0.02 | 0.02 | 0.02 | 0.02 | 0.02 |
| Powder component | Talc | | 35.00 | 35.00 | 35.00 | 35.00 | 35.00 | 35.00 | 30.00 |
| | Synthetic mica | | 35.00 | 35.00 | 35.00 | 35.00 | 35.00 | 35.00 | 30.00 |
| | Mica | | 3.38 | 3.38 | 3.38 | 3.38 | 3.38 | 11.88 | 1.88 |
| | Polymethyl methacrylate | | 1.00 | 1.00 | 1.00 | 1.00 | 1.00 | 1.00 | 1.00 |
| | Aluminum distearate | | 2.00 | 2.00 | 2.00 | 2.00 | 2.00 | 2.00 | 2.00 |
| | Red 226 | | 2.00 | 2.00 | 2.00 | 2.00 | 2.00 | 2.00 | 2.00 |
| | Yellow iron oxide | | 1.00 | 1.00 | 1.00 | 1.00 | 1.00 | 1.00 | 1.00 |
| | Titanium oxide-coated mica powder | | 7.00 | 7.00 | 7.00 | 7.00 | 7.00 | 7.00 | 7.00 |
| Volatile solvent | Light liquid isoparaffin | | 50 | 50 | 45 | 45 | 40 | 40 | 40 |
| Evaluation | Absence of powder flying | | B | A | A | A | A | B | A |
| | Picking up by applicator | | A | A | A | B | B | A | B |
| | Absence of caking | | A | A | A | B | B | A | B |
| | Feeling of use (spreading, adherence, good skin compatibility) | | B | A | A | B | B | B | A |
| | Good color development | | B | A | A | A | A | B | A |
| | Formability | | A | A | A | A | A | A | B |
| | Drop resistance | | A | A | A | A | A | B | A |

**[Table 2]**

| | | | Example | | | | | | |
|---|---|---|---|---|---|---|---|---|---|
| | | | 8 | 9 | 10 | 11 | 12 | 13 | 14 |
| Oily component | Component (A) | Silicone compound B | 4.00 | 4.00 | 4.00 | 4.00 | 4.00 | 4.00 | 4.00 |
| | Component (B) | Oil agent B | 3.50 | - | - | - | - | - | - |
| | | Oil agent C | - | 3.50 | - | - | - | - | - |
| | | Oil agent D | - | - | 3.50 | - | - | - | - |
| | | Oil agent E | - | - | - | 3.50 | - | - | - |
| | | Oil agent F | - | - | - | - | 3.50 | - | - |
| | | Oil agent G | - | - | - | - | - | 3.50 | - |
| | | Oil agent H | - | - | - | - | - | - | 3.50 |
| | Others | Ethylhexyl stearate | 6.00 | 6.00 | 6.00 | 6.00 | 6.00 | 6.00 | 6.00 |
| Other components | Sorbitan isostearate | | 0.10 | 0.10 | 0.10 | 0.10 | 0.10 | 0.10 | 0.10 |
| | Tocopherol | | 0.02 | 0.02 | 0.02 | 0.02 | 0.02 | 0.02 | 0.02 |
| Powder component | Talc | | 35.00 | 35.00 | 35.00 | 35.00 | 35.00 | 35.00 | 35.00 |
| | Synthetic mica | | 35.00 | 35.00 | 35.00 | 35.00 | 35.00 | 35.00 | 35.00 |
| | Mica | | 3.38 | 3.38 | 3.38 | 3.38 | 3.38 | 3.38 | 3.38 |
| | Polymethyl methacrylate | | 1.00 | 1.00 | 1.00 | 1.00 | 1.00 | 1.00 | 1.00 |
| | Aluminum distearate | | 2.00 | 2.00 | 2.00 | 2.00 | 2.00 | 2.00 | 2.00 |
| | Red 226 | | 2.00 | 2.00 | 2.00 | 2.00 | 2.00 | 2.00 | 2.00 |
| | Yellow iron oxide | | 1.00 | 1.00 | 1.00 | 1.00 | 1.00 | 1.00 | 1.00 |
| | Titanium oxide-coated mica powder | | 7.00 | 7.00 | 7.00 | 7.00 | 7.00 | 7.00 | 7.00 |
| Volatile solvent | Light liquid isoparaffin | | 50 | 50 | 50 | 50 | 50 | 50 | 50 |
| Evaluation | Absence of powder flying | | B | A | B | A | A | A | A |
| | Picking up by applicator | | A | A | A | B | C | B | B |
| | Absence of caking | | A | A | A | B | C | A | B |
| | Feeling of use (spreading, adherence, good skin compatibility) | | A | A | B | B | B | A | A |
| | Good color development | | A | A | A | A | A | A | A |
| | Formability | | A | A | A | A | A | A | A |
| | Drop resistance | | A | A | A | A | A | A | A |

**[Table 3]**

| | | | Example | | | |
|---|---|---|---|---|---|---|
| | | | 15 | 16 | 17 | 18 |
| Oily component | Component (A) | Silicone compound F | 4.00 | - | - | |
| | | Silicone compound G | - | 4.00 | - | |
| | | Silicone compound H | - | - | 4.00 | |
| | | Silicone compound I | - | - | - | 4.00 |
| | Component (B) | Oil agent A | 3.50 | 3.50 | 3.50 | 3.50 |
| | Others | Ethylhexyl stearate | 6.00 | 6.00 | 6.00 | 6.00 |
| Other components | Sorbitan isostearate | | 0.10 | 0.10 | 0.10 | 0.10 |
| | Tocopherol | | 0.02 | 0.02 | 0.02 | 0.02 |
| Powder component | Talc | | 35.00 | 35.00 | 35.00 | 35.00 |
| | Synthetic mica | | 35.00 | 35.00 | 35.00 | 35.00 |
| | Mica | | 3.38 | 3.38 | 3.38 | 3.38 |
| | Polymethyl methacrylate | | 1.00 | 1.00 | 1.00 | 1.00 |
| | Aluminum distearate | | 2.00 | 2.00 | 2.00 | 2.00 |
| | Red 226 | | 2.00 | 2.00 | 2.00 | 2.00 |
| | Yellow iron oxide | | 1.00 | 1.00 | 1.00 | 1.00 |
| | Titanium oxide-coated mica powder | | 7.00 | 7.00 | 7.00 | 7.00 |
| Volatile solvent | Light liquid isoparaffin | | 50 | 40 | 40 | 40 |
| Evaluation | Absence of powder flying | | B | B | A | A |
| | Picking up by applicator | | B | A | A | B |
| | Absence of caking | | A | A | A | B |
| | Feeling of use (spreading, adherence, good skin compatibility) | | B | B | A | B |
| | Good color development | | A | B | A | A |
| | Formability | | A | A | A | A |
| | Drop resistance | | A | A | A | A |

**[Table 4]**

| | | | Comparative Example | | | | | |
|---|---|---|---|---|---|---|---|---|
| | | | 1 | 2 | 3 | 4 | 5 | 6 |
| Oily component | Component (A) | Silicone compound B | - | - | - | 0.50 | 4.00 | 4.00 |
| | Component (B) | Oil agent A | 3.50 | 3.50 | 3.50 | 0.10 | 3.50 | - |
| | Others | Silicone compound J | - | 4.00 | - | - | - | - |
| | | Silicone compound K | - | - | 4.00 | - | - | - |
| | | Ethylhexyl stearate | 6.00 | 6.00 | 6.00 | 0.40 | 22.50 | 6.00 |
| | | Diisostearyl malate | 4.00 | - | - | - | - | 4.00 |
| Other components | Sorbitan isostearate | | 0.10 | 0.10 | 0.10 | 0.10 | 0.10 | 0.10 |
| | Tocopherol | | 0.02 | 0.02 | 0.02 | 0.02 | 0.02 | 0.02 |
| Powder component | Talc | | 35.00 | 35.00 | 35.00 | 40.00 | 25.00 | 35.00 |
| | Synthetic mica | | 35.00 | 35.00 | 35.00 | 40.00 | 30.00 | 35.00 |
| | Mica | | 3.38 | 3.38 | 3.38 | 5.88 | 1.88 | 3.38 |
| | Polymethyl methacrylate | | 1.00 | 1.00 | 1.00 | 1.00 | 1.00 | 1.00 |
| | Aluminum distearate | | 2.00 | 2.00 | 2.00 | 2.00 | 2.00 | 2.00 |
| | Red 226 | | 2.00 | 2.00 | 2.00 | 2.00 | 2.00 | 2.00 |
| | Yellow iron oxide | | 1.00 | 1.00 | 1.00 | 1.00 | 1.00 | 1.00 |
| | Titanium oxide-coated mica powder | | 7.00 | 7.00 | 7.00 | 7.00 | 7.00 | 7.00 |
| Volatile solvent | Light liquid isoparaffin | | 50 | 50 | 40 | 40 | 40 | 50 |
| Evaluation | Absence of powder flying | | D | D | A | D | A | D |
| | Picking up by applicator | | A | A | D | A | D | A |
| | Absence of caking | | A | A | D | A | D | A |
| | Feeling of use (spreading, adherence, good skin compatibility) | | C | C | B | C | C | C |
| | Good color development | | C | C | B | D | C | B |
| | Formability | | A | A | D | A | D | A |
| | Drop resistance | | B | C | A | D | A | D |

In Tables 1 to 4, the details of the respective components are as follows.

### [Oily component]

### Component (A)

Silicone compound A: Dimethyl polysiloxane (manufactured by Shin-Etsu Chemical Co., Ltd., KF-96-5,000cs, viscosity 4,900 mPa·s)
Silicone compound B: Dimethyl polysiloxane (manufactured by Shin-Etsu Chemical Co., Ltd., KF-96-10,000cs, viscosity 9,700 mPa·s)
Silicone compound C: Dimethyl polysiloxane (manufactured by Shin-Etsu Chemical Co., Ltd., KF-96-30,000cs, viscosity 29,000 mPa·s)
Silicone compound D: Dimethyl polysiloxane (manufactured by Shin-Etsu Chemical Co., Ltd., KF-96-50,000cs, Viscosity 49,000 mPa·s)
Silicone compound E: Dimethyl polysiloxane (manufactured by Shin-Etsu Chemical Co., Ltd., KF-96-100,000cs, viscosity 98,000 mPa·s)
Silicone compound F: Diphenylsiloxyphenyltrimethicone (manufactured by Shin-Etsu Chemical Co., Ltd., KF-54HV, viscosity 4,900 mPa·s)
Silicone compound G: Dimethiconol (manufactured by Shin-Etsu Chemical Co., Ltd., X-21-56613, viscosity 4,800 mPa·s)
Silicone compound H: Dimethiconol (manufactured by Shin-Etsu Chemical Co., Ltd., X-21-5666, viscosity 14,000 mPa·s)
Silicone compound I: Dimethiconol (manufactured by Shin-Etsu Chemical Co., Ltd., X-21-5849, viscosity 98,000 mPa·s)

### Component (B)

Oil agent A: Dipentaerythrityl hexa(hydroxystearate/stearate/rosinate) (manufactured by The Nisshin Oillio Group, Ltd., COSMOL 168ARV)
Oil agent B: Petrolatum (manufactured by Nikko Rica Corporation, SUNWHITE P-150)
Oil agent C: Hydroxyalkyl (C16-C18) hydroxydimer dilinoleyl ether (manufactured by Croda Japan K.K., SUPERMOL PS)
Oil agent D: Fatty acid (C10-30) (cholesterol/lanosterol) esters (manufactured by Croda Japan K.K., SUPER STEROL ESTER)
Oil agent E: (Phytosteryl/isostearyl/cetyl/stearyl/behenyl) dimer dilinoleate (manufactured by NIPPON FINE CHEMICAL CO., LTD., Plandool-G)
Oil agent F: Bis(behenyl/isostearyl/phytosteryl) dimer dilinoleyl dimer dilinolate (manufactured by NIPPON FINE CHEMICAL CO., LTD., Plandool-H)
Oil agent G: Dipentaerythrityl tetra(hydroxystearate/isostearate) (manufactured by The Nisshin Oillio Group, Ltd., COSMOL 168EV)
Oil agent H: Dipentaerythrityl hexa(hydroxystearate/stearate/rosinate) (manufactured by The Nisshin Oillio Group, Ltd., COSMOL 168M)

### (Others)

Silicone compound J: Dimethyl polysiloxane (manufactured by Shin-Etsu Chemical Co., Ltd., KF-96-1,000cs, viscosity 1,000 mPa·s)
Silicone compound K: Dimethyl polysiloxane (manufactured by Shin-Etsu chemical Co., Ltd., KF-96-300,000cs, viscosity 290,000 mPa·s)

### Incidentally, the viscosity of the silicone compound was measured, in a case in which the viscosity was 1,000 to 10,000 mPa·s, using a BH type viscometer and a rotor No. 3 at a speed of rotation of 10 rpm; in a case in which the viscosity was 10,000 to 100,000 mPa·s, the viscosity was measured using a BH type viscometer and a rotor No. 6 at a speed of rotation of 10 rpm; and in a case in which the viscosity was 100,000 to 800,000 mPa·s, the viscosity was measured using a BH type viscometer and a rotor No. 7 under the conditions of a speed of rotation of 4 rpm, at 25°C. Regarding the viscometer and the rotor, those manufactured by Toki Sangyo Co., Ltd. were used.

### [Powder component]

Talc: Average particle size 6.5 µm, aspect ratio 60
Synthetic mica: Average particle size 12 µm, aspect ratio 25
Mica: Average particle size 7.5 µm, aspect ratio 16
Polymethyl methacrylate: Spherical powder, average particle size 7.0 µm
Titanium oxide-coated mica powder: Average particle size 21.5 µm, aspect ratio 30

As shown in Tables 1 to 4, the solid powder cosmetics obtainable in Examples 1 to 11 and 13 to 18 were evaluated as "B" or "A" for the items of "Absence of powder flying", "Picking up by applicator", "Absence of caking", "Feeling of use (spreading, adherence, good skin compatibility)", "Good color development", "Formability", and "Drop resistance". The solid powder cosmetic obtainable in Example 12 was evaluated as "C" for the items of "Picking up by applicator" and "Absence of caking" but was evaluated as "B" or "A" for the other evaluation items.

### (Example 19: Eyeshadow)

| (Component) | (Blending proportion (mass%)) |
|---|---|
| 1. Silicone compound B | 6.00 |
| 2. Oil agent A | 3.00 |
| 3. Diphenylsiloxyphenyltrimethicone | 2.00 |
| 4. Dimethicone | 2.00 |
| 5. Sorbitan isostearate | 0.10 |
| 6. Tocopherol | 0.05 |
| 7. Talc | 20.00 |
| 8. Synthetic mica | 25.00 |
| 9. Mica | 5.35 |
| 10. Polymethyl methacrylate | 3.00 |
| 11. Aluminum distearate | 2.50 |
| 12. Yellow iron oxide | 3.00 |
| 13. Black iron oxide | 1.00 |
| 14. Colcothar | 2.00 |
| 15. Titanium oxide-coated mica powder | 25.00 |

Volatile solvent: Light liquid isoparaffin in 40 parts by mass with respect to 100 parts by mass of the sum of the above-described components 1 to 15

The details of the above-described components are similar to those already mentioned above.

### <Production method>

Components 7 to 15 were uniformly dispersed with a Henschel Mixer, and mixture I was obtained. Components 1 to 6 were heated to 70°C and uniformly dispersed, and mixture II was obtained. While mixture I was stirred with a Henschel Mixer, mixture II was added thereto, the mixture was uniformly dispersed, and a cosmetic base material was obtained. To 100 parts by mass of this cosmetic base material, 40 parts by mass of light liquid isoparaffin was added as a volatile solvent, the mixture was kneaded with a kneading machine, and thereby a slurry was obtained. The slurry thus obtained was charged into a metal tray and was compression-molded, and then the solvent was removed by drying the slurry. Thereby, a sample of an eyeshadow was produced.

### <Evaluation>

The sample of eyeshadow thus obtained was subjected to evaluations similar to those described above, and it was verified that the sample was evaluated as "A" for the items of "Absence of powder flying", "Picking up by applicator", "Absence of caking", "Feeling of use (spreading, adherence, good skin compatibility)", "Good color development", "Formability", and "Drop resistance".

### (Example 20: Facial powder)

| (Component) ( | (Blending proportion (mass%)) |
|---|---|
| 1. Silicone compound B | 1.00 |
| 2. Oil agent A | 2.00 |
| 3. Ethylhexyl stearate | 5.00 |
| 4. Sorbitan isostearate | 0.10 |
| 5. Tocopherol | 0.05 |
| 6. Talc | 50.00 |
| 7. Synthetic mica | 15.00 |
| 8. Mica | 7.35 |
| 9. Silica | 4.00 |
| 10. Aluminum distearate | 4.50 |
| 11. Yellow iron oxide | 0.80 |
| 12. Colcothar | 0.20 |
| 13. Titanium oxide-coated mica powder | 10.00 |

Volatile solvent: Light liquid isoparaffin in 50 parts by mass with respect to 100 parts by mass of the sum of the above-described components 1 to 13

The details of the above-described components are similar to those already mentioned above.

### <Production method>

Components 6 to 13 were uniformly dispersed with a Henschel Mixer, and mixture I was obtained. Components 1 to 5 were heated to 70°C and uniformly dispersed, and mixture II was obtained. While mixture I was stirred with a Henschel Mixer, mixture II was added thereto, the mixture was uniformly dispersed, and a cosmetic base material was obtained. To 100 parts by mass of this cosmetic base material, 50 parts by mass of light liquid isoparaffin was added as a volatile solvent, the mixture was kneaded with a kneading machine, and thereby a slurry was obtained. The slurry thus obtained was charged into a metal tray and was compression-molded, and then the solvent was removed by drying the slurry. Thereby, a sample of a facial powder was produced.

### <Evaluation>

The sample of facial powder thus obtained was subjected to evaluations similar to those described above, and it was verified that the sample was evaluated as "A" for the items of "Absence of powder flying", "Picking up by applicator", "Absence of caking", "Feeling of use (spreading, adherence, good skin compatibility)", "Good color development", "Formability", and "Drop resistance".

### (Example 21: Foundation)

| (Component) ( | (Blending proportion (mass%)) |
|---|---|
| 1. Silicone compound B | 1.00 |
| 2. Oil agent A | 0.50 |
| 3. Diphenylsiloxyphenyltrimethicone | 3.00 |
| 4. Dimethicone | 2.00 |
| 5. Sorbitan isostearate | 1.00 |
| 6. Tocopherol | 0.05 |
| 7. Talc | 20.00 |
| 8. Synthetic mica | 35.00 |
| 9. Mica | 2.95 |
| 10. Boron nitride | 25.00 |
| 11. Silica | 3.00 |
| 12. Aluminum distearate | 1.50 |
| 13. Yellow iron oxide | 1.00 |
| 14. Colcothar | 0.30 |
| 15. Titanium oxide | 0.70 |
| 16. Titanium oxide-coated mica powder | 3.00 |

As a volatile solvent, light liquid isoparaffin was mixed in an amount of 50 parts by mass with respect to 100 parts by mass of the sum of the above-described components 1 to 16.

The details of the above-described components are similar to those already mentioned above.

### <Production method>

Components 7 to 16 were uniformly dispersed with a Henschel mixer, and mixture I was obtained. Components 1 to 6 were heated to 70°C and uniformly dispersed, and mixture II was obtained. While mixture I was stirred with a Henschel mixer, mixture II was added thereto, the mixture was uniformly dispersed, and a cosmetic base material was obtained. To 100 parts by mass of this cosmetic base material, 50 parts by mass of light liquid isoparaffin was added as a volatile solvent, the mixture was kneaded with a kneading machine, and thereby a slurry was obtained. The slurry thus obtained was charged into a metal tray and was compression-molded, and then the solvent was removed by drying the slurry. Thereby, a sample of a foundation was produced.

### <Evaluation>

The sample of foundation thus obtained was subjected to evaluations similar to those described above, and it was verified that the sample was evaluated as "A" for the items of "Absence of powder flying", "Picking up by applicator", "Absence of caking", "Feeling of use (spreading, adherence, good skin compatibility)", "Good color development", "Formability", and "Drop resistance".

### (Example 22: Eyebrow pencil)

| (Component) | (Blending proportion (mass%)) |
|---|---|
| 1. Silicone compound B | 2.00 |
| 2. Oil agent A | 1.50 |
| 3. Diphenylsiloxyphenyltrimethicone | 10.00 |
| 4. Sorbitan isostearate | 0.50 |
| 5. Tocopherol | 0.05 |
| 6. Talc | 60.00 |
| 7. Synthetic mica | 10.00 |
| 8. Mica | 4.95 |
| 9. Silica | 1.00 |
| 10. Yellow iron oxide | 5.00 |
| 11. Black iron oxide | 2.00 |
| 12. Titanium oxide | 3.00 |

As a volatile solvent, light liquid isoparaffin was mixed in an amount of 40 parts by mass with respect to 100 parts by mass of the sum of the above-described components 1 to 12.

The details of the above-described components are similar to those already mentioned above.

### <Production method>

Components 6 to 12 were uniformly dispersed with a Henschel Mixer, and mixture I was obtained. Components 1 to 5 were heated to 70°C and uniformly dispersed, and mixture II was obtained. While mixture I was stirred with a Henschel Mixer, mixture II was added thereto, the mixture was uniformly dispersed, and a cosmetic base material was obtained. To 100 parts by mass of this cosmetic base material, 40 parts by mass of light liquid isoparaffin was added as a volatile solvent, the mixture was kneaded with a kneading machine, and thereby a slurry was obtained. The slurry thus obtained was charged into a metal tray and was compression-molded, and then the solvent was removed by drying the slurry. Thereby, a sample of an eyebrow pencil was produced.

### <Evaluation>

The sample of eyebrow pencil thus obtained was subjected to evaluations similar to those described above, and it was verified that the sample was evaluated as "A" for the items of "Absence of powder flying", "Picking up by applicator", "Absence of caking", "Feeling of use (spreading, adherence, good skin compatibility)", "Good color development", "Formability", and "Drop resistance".

### (Example 23: Blusher)

| (Component) | (Blending proportion (mass%)) |
|---|---|
| 1. Silicone compound B | 1.50 |
| 2. Oil agent A | 1.00 |
| 3. Ethylhexyl stearate | 3.00 |
| 4. Sorbitan isostearate | 0.01 |
| 5. Tocopherol | 0.05 |
| 6. Talc | 40.00 |
| 7. Synthetic mica | 40.00 |
| 8. Mica | 4.44 |
| 9. Polymethyl methacrylate | 1.00 |
| 10. Aluminum distearate | 2.00 |
| 11. Red 226 | 2.00 |
| 12. Titanium oxide-coated mica powder | 5.00 |

The details of the above-described components are similar to those already mentioned above.

### <Production method>

Components 6 to 12 were uniformly dispersed with a Henschel Mixer, and mixture I of powder components was obtained. Components 1 to 5 were heated to 70°C and uniformly dispersed, and mixture II of oily components was obtained. While mixture I was stirred with a Henschel Mixer, mixture II was added thereto, and the mixture was uniformly dispersed. This dispersion was charged into a metal tray and was compression-molded, and thereby a sample of a blusher was produced.

### <Evaluation>

The sample of blusher thus obtained was subjected to evaluations similar to those described above, and it was verified that the sample was evaluated as "A" for the items of "Absence of powder flying", "Picking up by applicator", "Feeling of use (spreading, adherence, good skin compatibility)", "Good color development", "Formability", and "Drop resistance", and was evaluated as "B" for the item "Absence of caking".

### Industrial Applicability

According to the present invention, a solid powder cosmetic which has satisfactory feeling of use and sufficient drop resistance, in which caking is sufficiently suppressed and picking up by an applicator is satisfactory, and which can sufficiently suppress powder flying, can be provided.

## Claims

1. A solid powder cosmetic, containing a powder component and an oily component,
wherein the content of the oily component is 5% to 25% by mass based on the total amount of the solid powder cosmetic, and
the oily component includes: (A) a silicone compound whose viscosity at 25°C is 4,000 to 100,000 mPa·s; and (B) an oil agent that is in a semi-solid state at 25°C.

2. The solid powder cosmetic according to claim 1, wherein the component (A) includes one or more kinds of silicone compounds selected from the group consisting of dimethyl polysiloxane, diphenyl polysiloxane, and dimethiconol.

3. The solid powder cosmetic according to claim 1 or 2, wherein the component (B) includes one or more kinds of oil agents selected from the group consisting of a pentaerythritol fatty acid ester, petrolatum, a dimer acid ester, a dimer diol derivative, a phytosterol fatty acid ester, and a cholesterol fatty acid ester.

4. The solid powder cosmetic according to any one of claims 1 to 3, wherein the content of the component (A) is 0.5% to 15.0% by mass based on the total amount of the solid powder cosmetic, and
the content of the component (B) is 0.1% to 10.0% by mass based on the total amount of the solid powder cosmetic.
